# EUROPEAN PATENT APPLICATION

(11) **EP 3 358 484 A1**
(43) Date of publication of application: **08.08.2018**
(21) Application number: 17801317.3
(22) Date of filing: 06.04.2017
(51) Int. Cl.: G06F 19/00

(54) **DATA PRESENTING METHOD AND DEVICE, TERMINAL, AND STORAGE MEDIUM**

(30) Priority: 30.06.2016 CN 201610505348
(71) Applicant: Ping An Technology (Shenzhen) Co., Ltd., Shenzhen, Guangdong 518000 (CN)
(72) Inventor: ZHENG, Jiaer, Shenzhen Guangdong 518000 (CN); XU, Liang, Shenzhen Guangdong 518000 (CN)
(74) Representative: Michalski Hüttermann & Partner Patentanwälte mbB
(86) International application number: PCT/CN2017/079620
(87) International publication number: WO 2018/000884

(57) **Abstract**

A method of data displaying, which includes: acquiring an information displaying instruction comprising a displaying manner; when the displaying manner is a first type displaying manner, acquiring a first data to be processed, wherein the first data to be processed comprises treatment information in a plurality of dimensions, a dimension sign corresponding to the treatment information, and associated relations between treatment information in different dimensions, wherein each dimension comprises at least one of treatment information; generating a nodal profile according to the treatment information and the associated relations between the treatment information in different dimensions, recording the one-to-one correspondence relation between various nodes in the nodal profile and the treatment information, wherein the connection relations between various nodes correspond to the associated relations between the treatment information in different dimensions; acquiring a dimension sign corresponding to each node according to the one-to-one correspondence relation between various nodes in the nodal profile and the dimension sign corresponding to the treatment information; and adding a corresponding tag information to each node according to a preset correspondence relation between the dimension sign and the tag information, generating a treatment network diagram, and displaying the treatment network diagram.

## Description

This application claims priority to Chinese Patent Application No. 2016105053485, entitled "METHOD AND APPARATUS OF DATA DISPLAYING" filed on June 30, 2016, the contents of which is expressly incorporated by reference herein in its entirety.

### FIELD OF THE INVENTION

The present disclosure relates to the field of data processing technology, and in particular relates to a method, an apparatus, a terminal, and a storage medium of data displaying.

### BACKGROUND OF THE INVENTION

With the improvement of social insurance, certain medicine costs and certain fees incurred in the treatment can be covered with a social insurance card. As such, various fraud uses of social insurance cards keep on happening, such as asking the physician to prescribe some expensive medicines which are resold to medicine dealers for profits or, for example, forcing the way to be hospitalized to enjoy coverage and so on. As such, related organizations usually will analyze the treatment information of the social insurance card to acquire suspiciously problematic medical information and investigate the same to prevent and identify such unjust way of profit making.

As the data volume is enormous and people are usually insensible to pure numbers or words, a conventional way is to display the social insurance treatment information by way of basic charts and diagrams, such as a broken line chart that reflects the trend of the amount.

However, such kind of conventional visualized charts/diagrams only allow displaying a single dimension at the same time, some critical implicit information cannot be demonstrated in the charts/diagrams, the information content is constrained.

### SUMMARY OF THE INVENTION

According to various embodiments, a method, an apparatus, a terminal, and a storage medium of data displaying are provided.

A method of data displaying includes:
acquiring an information displaying instruction which includes a displaying manner;
acquiring a first data to be processed when the displaying manner is a first type displaying manner, the first data to be processed includes treatment information in a plurality of dimensions, the dimension sign corresponding to the treatment information, and the associated relations between the treatment information in different dimensions, each dimension includes at least one of treatment information;
generating a nodal profile according to the treatment information and the associated relations between the treatment information in different dimensions, recording the one-to-one correspondence relation between various nodes in the nodal profile and the treatment information, connection relations between various nodes correspond to the associated relations between the treatment information in different dimensions;
acquiring a dimension sign corresponding to each node according to the one-to-one correspondence relation between various nodes in the nodal profile and the dimension sign corresponding to the treatment information; and
according to a preset correspondence relation between the dimension sign and the tag information, adding corresponding tag information to each node, generating a treatment network diagram, and displaying the treatment network diagram.

An apparatus of data displaying includes:
an acquiring unit configured to acquire an information displaying instruction which includes a displaying manner;
the acquiring unit is further configured to acquire a first data to be processed when the displaying manner is a first type displaying manner, the first data to be processed includes treatment information in a plurality of dimensions, a dimension sign corresponding to the treatment information, and associated relations between the treatment information in different dimensions, each dimension includes at least one of treatment information;
a graph generating unit configured to generate a nodal profile according to the treatment information and the associated relations between the treatment information in different dimensions, recording the one-to-one correspondence relation between various nodes in the nodal profile and the treatment information, connection relations between various nodes correspond to the associated relations between the treatment information in different dimensions;
a corresponding dimension determining unit configured to acquire a dimension sign corresponding to each node according to the one-to-one correspondence relation between various nodes in the nodal profile and the dimension sign corresponding to the treatment information;
a tag processing unit configured to add corresponding tag information to each node according to a preset correspondence relation between the dimension sign and the tag information, generate a treatment network diagram; and
a displaying unit configured to display the treatment network diagram.

A terminal including a processor; and a memory storing instructions, which, when executed by the processor cause the processor to perform steps including:
acquiring an information displaying instruction which includes a displaying manner;
acquiring a first data to be processed when the displaying manner is a first type displaying manner, the first data to be processed includes treatment information in a plurality of dimensions, the dimension sign corresponding to the treatment information, and the associated relations between the treatment information in different dimensions, each dimension includes at least one of treatment information;
generating a nodal profile according to the treatment information and the associated relations between the treatment information in different dimensions, recording the one-to-one correspondence relation between various nodes in the nodal profile and the treatment information, connection relations between various nodes correspond to the associated relations between the treatment information in different dimensions;
acquiring a dimension sign corresponding to each node according to the one-to-one correspondence relation between various nodes in the nodal profile and the dimension sign corresponding to the treatment information; and
according to a preset correspondence relation between the dimension sign and the tag information, adding corresponding tag information to each node, generating a treatment network diagram, and displaying the treatment network diagram.
at least one non-volatile computer readable storage medium storing computer readable instructions, and at least one processor that execute the instructions to provide:
   acquiring an information displaying instruction which includes a displaying manner;
   acquiring a first data to be processed when the displaying manner is a first type displaying manner, the first data to be processed includes treatment information in a plurality of dimensions, the dimension sign corresponding to the treatment information, and the associated relations between the treatment information in different dimensions, each dimension includes at least one of treatment information;
   generating a nodal profile according to the treatment information and the associated relations between the treatment information in different dimensions, recording the one-to-one correspondence relation between various nodes in the nodal profile and the treatment information, connection relations between various nodes correspond to the associated relations between the treatment information in different dimensions;
   acquiring a dimension sign corresponding to each node according to the one-to-one correspondence relation between various nodes in the nodal profile and the dimension sign corresponding to the treatment information; and
   according to a preset correspondence relation between the dimension sign and the tag information, adding corresponding tag information to each node, generating a treatment network diagram, and displaying the treatment network diagram.

The details of at least one embodiments of the present disclosure will be described with reference to the following drawings and description. Other characteristic, purposes and advantages of the present disclosure will be more apparent from the specification, drawing and claims.

### BRIEF DESCRIPTION OF THE DRAWINGS

To illustrate the technical solutions according to the embodiments of the present invention or in the prior art more clearly, the accompanying drawings for describing the embodiments or the prior art are introduced briefly in the following. Apparently, the accompanying drawings in the following description are only some embodiments of the present invention, and persons of ordinary skill in the art can derive other drawings from the accompanying drawings without creative efforts..
FIG. 1 is a block diagram of a terminal according to an embodiment;
FIG. 2 is a flowchart of a method of data displaying according to an embodiment;
FIG. 3 is a schematic structure diagram of a treatment network according to an embodiment;
FIG. 4 is a flowchart of a method of infographic displaying of the dimension of geography according to an embodiment;
FIG. 5 is a schematic view of the sub-diagram of the treatment network according to an embodiment;
FIG. 6 is a block diagram of an apparatus of data displaying according to an embodiment; and
FIG. 7 is a block diagram of an apparatus of data displaying according to another embodiment.

### DETAILED DESCRIPTION OF THE EMBODIMENTS

The present disclosure will be described in details in combination with the accompanying drawings and embodiments such that the purpose, technical solution and advantages of the present disclosure will be more apparent. It should be understood that the particular embodiments are described for the purpose of illustrating as opposed to restricting the present invention.

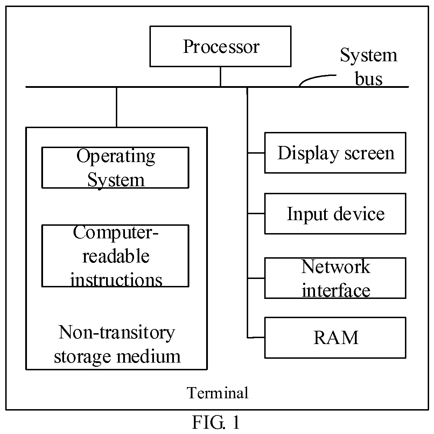

In an embodiment, a terminal is provided whereon applications can be installed. Such terminal can be a mobile phone, a tablet, a notebook computer or a desktop computer and so on. As shown in FIG. 1, the terminal includes a processor connected through the system bus, a RAM(Random Access Memory), a non-transitory storage medium, a network interface, a display screen and an input device. The processor is configured to provide computation and control capability to support the operation of the entire terminal. An operating system and computer-readable instructions are stored in the non-transitory storage medium of the terminal, the computer-readable instructions can be executed by the processor to implement the method of data displaying according to the embodiments as follows. The RAM of the terminal provides an operation environment for the operating system and the computer-readable instructions in the non-transitory storage medium. The network interface is configured to be connected to the network for communication. The display screen of the terminal can be a liquid crystal display screen or an electronic ink and so on, in the present embodiment, the display screen can be the output device of the terminal for displaying various interfaces, such as for displaying treatment network diagram. The input device can be a touch layer covered on the display screen, a button, a trackball or a touch pad configured on the shell of an electronic device, or an external keyboard, touch pad or mouse and so on for the user to input various commanding instructions, for example, in the present embodiment, for the user to input an information display instruction.

A person skilled in the art should understand, FIG. 1 is exemplary block diagram to show the structure of the terminal in accordance with an embodiment of the present disclosure and does not limit the terminal to the embodiment; in other embodiments, compared with the structure shown in FIG. 1, the particular terminal may include more or less components, be configured with other components not shown in FIG. 1, or have a different configuration.

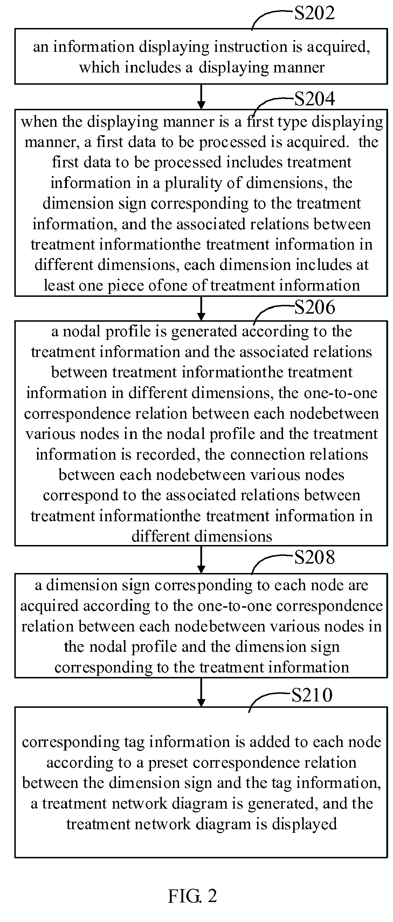

As shown in FIG. 2, in an embodiment, a method of data displaying is provided, the method will be described as being applied on the terminal illustrated in FIG. 1, the method specifically includes the steps as follows:

In step S202, an information displaying instruction is acquired, which includes a displaying manner.

In step S204, when the displaying manner is a first type displaying manner, a first data to be processed is acquired. the first data to be processed includes treatment information in a plurality of dimensions, the dimension sign corresponding to the treatment information, and the associated relations between the treatment information in different dimensions, each dimension includes at least one of treatment information.

The displaying manner includes a first type display manner and/or a second type display manner. When acquiring the information displaying instruction, it is determined which type of displaying manner the displaying manner is in the information displaying instruction, so as to correspondingly acquire the data to be processed required for the displaying manner of such type. In an embodiment, the first type displaying manner includes the manner of displaying by way of a treatment network diagram, the second type displaying manner includes the manner of displaying by way of an infographic in geography dimension.

It should be understood that, the displaying manner in the information displaying instruction can either be the first type displaying manner or the second type displaying manner or both the first type displaying manner and the second type displaying manner at the same time. The particular configuration can be determined in view of the needs of the business. Furthermore, when the displaying manner includes the first type displaying manner and the second type displaying manner at the same time, data to be processed respectively required for each type of displaying manner can be acquired correspondingly, and corresponding displaying graph are generated.

When the displaying manner included in the information displaying instruction is a first type displaying manner, the first data to be processed is acquired. The first data to be processed is the parameters required by the graph satisfying the first type displaying manner.

In particular, the first data to be processed includes treatment information in a plurality of dimensions, the dimension sign corresponding to the treatment information, and the associated relations between the treatment information in different dimensions. Each dimension includes at least one of treatment information. The treatment information refers to the series of information related to the treatment and generated in the treatment process. For example, the patient, the hospital, the physician, the date, the disease, the purchased medicine and other treatment information.

Furthermore, the plurality of dimensions include at least two of the followings: a user dimension, a medicine dimension, a medical program dimension, a hospital dimension, a disease dimension and a doctor dimension. In an embodiment, the plurality of dimensions includes a user dimension and at least another dimension in addition to the user dimension. It should be understood that the number and types of dimensions required can be preset in view of the actual situation.

Accordingly, the treatment information in the user dimension includes treatment user information, the treatment information in the medicine dimension includes information of medicine purchased during treatment, the treatment information in the hospital dimension includes treatment hospital information, the treatment information in the disease dimension includes the treatment disease information, and the treatment information in the doctor dimension includes treatment doctor information.

It should be understood that the dimension sign corresponding to the treatment information is the dimension sign of the dimension where the treatment information belongs. For instance, the dimension sign corresponding to the treatment user information is the user dimension sign, the information of the medicine purchased in treatment is the medicine dimension sign.

Furthermore, the associated relation between the treatment information of difference dimensions refers to visiting, purchasing, diagnosing, suffering and other underlying relations between different dimensions. For example, a user A visited a hospital B for a physician C to diagnose a disease D, the physician C prescribed a medicine E on the disease D, and the user A purchased the medicine E. Herein, the user A visited the hospital B and the physician C, the user A has the disease D and purchased the medicine E, as such, the user A, the hospital B, the physician C, the disease D and the medicine E have associated relations. Where the physician C diagnoses the disease D, the physician C therefore has an associated relation with the disease D. The medicine E is prescribed for the disease D, thus the disease D and the medicine E has an associated relation. It should be understood that the definition of associated relation can be set according to the actual needs, and associated relations between particular treatment information are not defined and limited herein.

Examples are herein provided to describe each dimension with at least one (i.e., one or more) treatment information. For instance, the user dimension can include one user information of user A, or several user information of user A, B and C. The hospital dimension can include at least one hospital information.

In step S206, a nodal profile is generated according to the treatment information and the associated relations between the treatment information in different dimensions, the one-to-one correspondence relation between various nodes in the nodal profile and the treatment information is recorded, the connection relations between various nodes correspond to the associated relations between the treatment information in different dimensions.

In particular, a nodal profile is generated according to the treatment information and the associated relations between the treatment information in different dimensions. The each node in the nodal profile is correspondent to the treatment information on an one-to-one basis, the connection relations between various nodes correspond to the associated relations between the treatment information in different dimensions. Further, the nodes can be named after the title of the corresponding treatment information.

For example, the different dimensions include the six dimensions of the user dimension, hospital dimension, disease dimension, medical project dimension and medicine dimension. The user dimension includes user A and user B, the hospital dimension includes hospital C, the doctor dimension includes doctor D, the disease dimension includes disease E, the medical project dimension includes medical project F, medicine dimension includes medicine G and H. User A has associated relations with C, D, E, F and G respectively, user B has associated relations with C, D, E, F and H respectively, hospital C and doctor D have an associated relation, doctor D and disease E have an associated relation, disease E and medicine G and H have an associated relation.

As such, nodes are respectively generated based on A, B, C, D, E, F, G and H, and treatment information and corresponding nodes are connected according to the associated relations between different treatment information of different dimensions. Based on the foregoing examples, a node corresponding to A is connected to a node corresponding to C, D, E, F and G on an one-to-one basis, a node corresponding to B is connected to a node corresponding to C, D, E, F and H on a one-to-one basis, and to respective corresponding nodes of C and D, of D and E, and of E and G and H are connected. It should be understood, that inter-nodes connection can be performed by way of a straight line, a curved line or other connections.

Additionally, corresponding connection transit nodes can be further introduced respectively for dimension types to be connected by each node, a node corresponding to the type of dimension corresponding to the node are transit connected. For instance, node A is disease A, and is to be connected to several doctors respectively in the doctor dimension and several medicine nodes in the medicine dimension, then connections are all started from node A to different doctors and medicines, which will cause the lines to be too concentrated and is conductive for identification. As such, transit nodes X and Y can be introduced, whereby node A is connected to the transit node X and Y, and transit node X is connected to different doctor nodes, and transit node Y is connected to different medicine nodes. The connection form is not limited thereto.

Furthermore, the one-to-one correspondence relation between different nodes and the treatment information is recorded.

In step S208, a dimension sign corresponding to each node are acquired according to the one-to-one correspondence relation between various nodes in the nodal profile and the dimension sign corresponding to the treatment information.

In particular, the dimension sign corresponding to each node are acquired according to the one-to-one correspondence relation between various nodes in the nodal profile and the dimension sign corresponding to the treatment information included in the acquired first data to be processed, such that the dimension sign corresponding to each node in the nodal profile are acquired. For example, the dimension sign corresponding to the node corresponding to user A is the user dimension sign.

In step S210, corresponding tag information is added to each node according to a preset correspondence relation between the dimension sign and the tag information, a treatment network diagram is generated, and the treatment network diagram is displayed.

In the present embodiment, the correspondence between the dimension sign and the sign information is preset, different dimension signs corresponds to different sign information. Furthermore, the sign information can be a color information, a shape information or other forms of sign. In the embodiment, the particular form of the sign information is not limited thereto, as long as the particular form can distinguish the signs.

Furthermore, according to the corresponding relation and the dimension sign corresponding to each node acquired in step S208, the sign information corresponding to each node can be acquired, corresponding sign information is further added to each node, thereby generating and displaying the treatment network diagram.

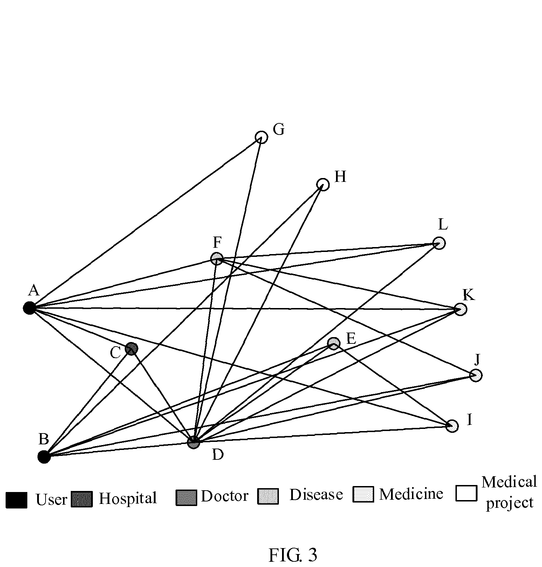

The treatment network diagram is described in combination with FIG. 3. The treatment network diagram illustrated in FIG. 3 includes nodes A, B, C, D, E, F, G, H, I, J, K and L (the node name is the name of the treatment information) and the connection relations between various nodes. Nodes A and B are corresponding to user dimension sign, C is corresponding hospital dimension sign, D is corresponding to the doctor dimension sign, E and F are corresponding to the disease dimension sign, G and H are corresponding to the medical project dimension sign, and I, J, K and L are corresponding to the medicine dimension sign. Different dimension signs are corresponding to different color tags, as such, each node is respectively tagged with a corresponding color, therein, a node corresponding to the same dimension sign are corresponding to the same color. For example, both nodes A and B are corresponding to the user dimension sign, thus both are of the same color. The connection relations between various nodes represents that the treatment information corresponding to the two nodes has an associated relation, for example, as node A and node K are connected, it suggests that the user A corresponding to node A and the medicine K corresponding to node K has an associated relation that, for example, user A purchased medicine K.

In the present embodiment, the information displaying instruction is acquired, which includes the displaying manners. When the displaying manner is a first type displaying manner, the first data to be processed is acquired which includes treatment information in various dimensions, a dimension sign corresponding to the treatment information and the associated relations between the treatment information in different dimensions, each dimension includes at least one of treatment information; a nodal profile is generated according to the treatment information and the associated relations between the treatment information in different dimensions, the one-to-one correspondence relation between various nodes in the nodal profile and the treatment information is recorded, the connection relations between various nodes correspond to the associated relations between the treatment information in different dimensions; the a dimension sign corresponding to each node are acquired according to the one-to-one correspondence relation between various nodes in the nodal profile and the treatment information and the a dimension sign corresponding to the treatment information; according to the preset correspondence between the dimension sign and the tag information, each node is correspondingly added with tag information and a treatment network diagram is generated and displayed. Treatment information in several dimensions are reflected at the same time via the treatment network diagram, thereby improving the amount of information reflected in the diagram.

Besides, the treatment network diagram that reflects multi-dimension and large number of information can further reflect some key implicit information, thereby improving the finding of suspiciously problematic treatment information.

In an embodiment, the treatment network diagram is dynamic, a dragging or a zoom-in/out instruction can be detected, the position of the node or the relative position between nodes is correspondingly modified according to the dragging instruction, or the corresponding node information in the treatment network diagram is correspondingly zoomed out or zoomed in according to the zoom-in/out instruction. Furthermore, the degree of detail of the information displayed after zoom out or zoom in can be different, for example, the node position, relative position between nodes and the connection relation between nodes can be displayed after zoom out, and node name and other details can be further displayed in addition to the node position, relative position between nodes and the connection relation between nodes after zoom in. The particularity of the displaying information can be determined by the extent of the zooming.

In an embodiment, the first data to be processed also includes the number of different treatment information, the method further includes: adjusting correspondingly the visibility properties of the node in the treatment network diagram and corresponding to the treatment information according to the number of treatment information, the visibility properties refer to properties that can be perceptually demonstrated.

It should be understood that in the first treatment of the user, a first treatment record will be generated, a treatment information includes a series of treatment information in various dimensions, as such, when there are several treatment processes, there may be several records of the same treatment information. For example, when user A is treated by doctor B for a first time and again for another time, the number corresponding to doctor B will be two.

In the present embodiment, the first data to be processed further includes the number of treatment information; The visibility properties of the node in the treatment network diagram and corresponding to the treatment information are adjusted according to the number of treatment information. The visibility properties refer to properties that can be perceptually demonstrated, including the size, the color and other visible properties of the node.

In particular, the visibility properties of the node can be adjusted by, based on the preset correspondence between the visibility property values and the treatment information number, determining the range value of the number corresponding to each treatment information, and then determining the visibility property value of the node corresponding to the range value of the number. Alternatively, the ratio relation of the treatment information number over the visibility property value of the node can be preset, whereby the visibility property of the node can be adjusted. Also, the ratio relation can be a preset constant ratio, or a ratio formula that is dynamic and changeable. Furthermore, with respect to each different dimensions, different correspondences between the visibility property value of a node and the treatment information number range or different ratio relations of treatment information number and visibility properties of the node can be configured. The embodiment of the disclosure is not limited in the respect, as long as the visibility properties of the node corresponding to the treatment information are adjusted according to the number of treatment information.

In the present embodiment, the visibility properties of the node in the treatment network diagram and corresponding to the treatment information are adjusted according to the number of treatment information, the amount of information reflected in the treatment network diagram is further improved. Besides, by adjusting the visibility property of the node, the difference of each treatment information can be reflected more intuitively, thereby further accelerating the finding of suspiciously problematic treatment information.

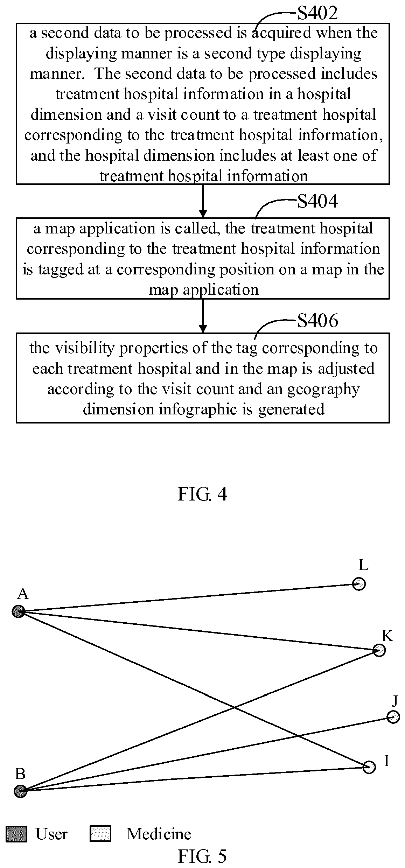

As shown in FIG. 4, in an embodiment, the method further includes a step of generating geography dimension infographic, which particularly includes the steps as follows:
In step S402, a second data to be processed is acquired when the displaying manner is a second type displaying manner. The second data to be processed includes treatment hospital information in a hospital dimension and a visit count to a treatment hospital corresponding to the treatment hospital information, and the hospital dimension includes at least one of treatment hospital information.

In the present embodiment, when the displaying manner is a second type display manner, a second data to be processed is acquired. The second data to be processed is the parameters required by the graph satisfying the second type displaying manner.

In particular, the second data to be processed includes treatment hospital information in a hospital dimension and a visit count to a treatment hospital corresponding to the treatment hospital information. The hospital dimension includes at least one of treatment hospital information.

Further, the treatment hospital information includes the name of the treatment hospital, and can also include the address of the treatment hospital.

In step S404, a map application is called, the treatment hospital corresponding to the treatment hospital information is tagged at a corresponding position on a map in the map application.

In particular, a map application is called, the longitude and latitude of the treatment hospital is queried according to the treatment hospital information, such as the name or address or other information of the treatment hospital, and the treatment hospital is tagged at a corresponding position on a map in the map application.

In step S406, the visibility properties of the tag corresponding to each treatment hospital and in the map is adjusted according to the visit count and an geography dimension infographic is generated.

Further, the visibility properties of the tag corresponding to each treatment hospital and in the map is adjusted according to the visit count. The visibility properties refer to properties that can be perceptually demonstrated, such as the color, size, shape and the tag.

In particular, based on the preset correspondence between the visibility property values of the tag and the preset visit count range value to the treatment hospital, the visit count range value corresponding to the visit count to each treatment hospital is determined, and then the visibility property value of the node corresponding to the visit count range value is determined according to the correspondence, based on the visibility properties of such tag, the visibility properties of the node can be adjusted and a geography dimension infographic is generated. Further, the generated geography dimension infographic is displayed.

Alternatively, the ratio relation of the visit count of the treatment hospital over the visibility property value of the node can be preset, whereby the visibility property of the node can be adjusted. Ways to adjust the visibility property based on the visit count of the treatment hospital are not enumerated herein, as long as such way can adjust the visibility properties of the tag corresponding to the visit count of the treatment hospital.

For example, the size of the tag can be adjusted based on the visit count to the treatment hospital. When the tag is a circle, the correspondence between the visit count range value and the radius value of the tag can be preset, such as, when the visit count is from 1 to 2, the corresponding tag radius value is 2, when the visit count is from 3 to 5, the corresponding tag radius value is 3, when the visit count is from 6 to 10, the corresponding tag radius value is 6. As such, the treatment hospital in the hospital dimension in the second data to be processed acquired is hospital A and hospital B, the visit count to hospital A is 2, the visit count to the hospital B is 8, thus the corresponding tag radius value of hospital A on a map in the map application is 2, and the corresponding tag radius value of hospital B on a map in the map application is 6.

Further, the corresponding tag in the map of the treatment hospital can include the name of the treatment hospital.

In the present embodiment, when the displaying manner is a second type displaying manner, the treatment hospital information in a hospital dimension and the visit count to a treatment hospital corresponding to the treatment hospital information in the second data to be processed are acquired, the hospital dimension includes at least one of treatment hospital information. The treatment hospital corresponding to the treatment hospital information is tagged on a map in the map application at a corresponding position. The visibility properties of the tag corresponding to the treatment hospital and in the map are adjusted according to the visit count to the treatment hospital, and a geography dimension infographic is generated. By integrating the hospital dimension and the geography dimension, the treatment behavior profile can be displayed intuitively, thereby improving the finding of suspiciously problematic treatment information.

In an embodiment, the second data to be processed also includes the visiting date to each treatment hospital, after the generating of the geography dimension infographic, further includes a step of integrating the visiting dates to each treatment hospital, and successively tagging, one over another, the visiting dates to each treatment hospital at a corresponding position of the treatment hospital in the geography dimension infographic dynamically according to the time sequential order of the integrated visiting dates.

In the present embodiment, the second data to be processed further includes the number of visiting dates to each treatment hospital. After the geography dimension infographic is generated, the visiting dates of each treatment hospital are integrated, i.e., to sequence the visiting dates of each treatment hospital in unity.

Further, according to sequential order of the integrated visiting dates, the visited treatment hospital corresponding to each visiting date is sequentially found, and an overlapping tag of the visiting date at the corresponding position in the geography dimension infographic of the corresponding visited hospital is added. It should be understood, after finishing the overlapping tagging of the last visiting date, the finding of the treatment hospital corresponding to the next visit date, and the overlapping tagging at the corresponding position in the geography dimension infographic are continued. So as to effect the integrated information dynamic graph of the hospital dimension, the geography dimension and the time dimension.

Such as, the treatment hospital in the hospital dimension includes Y1 and Y2, the visit dates to Y1 are January 1st and 3rd, the visit dates to Y2 are January 2nd, 4th and 5th. Then it will be found that the corresponding treatment hospital of date January 1st is Y1, it is overlapping tagged on the corresponding position in the geography dimension infographic and of the treatment hospital Y1 that the visit date is January 1st. Next, it continued to find the corresponding treatment hospital corresponding to the date January 2nd is Y2, and it is overlapping tagged on the corresponding position in the geography dimension infographic and of the treatment hospital Y2 that the visit date is January 2nd, and so on, a dynamic and overlapping tagging of the visiting time is thus effected. Thereby implementing the integrated information dynamic graph of the hospital dimension, the geography dimension and the time dimension.

In the present embodiment, the time dimension, the hospital dimension and the geography dimension are integrated to further improve the amount of information to be reflected in the graph to more intuitively reflect the implicit key information between multi-dimension information, so as to accelerate the finding of the problematic treatment information.

In an embodiment, the method further includes: a screening and viewing instruction is received, a dimension sign corresponding to the screening and viewing instruction is acquired, corresponding nodes and the connection relation between the acquired nodes are acquired from the treatment network sub-diagram, and a treatment network sub-diagram according to the acquired nodes and connection relations.

In the present embodiment, the screening and viewing instruction is received, which includes the viewing type sign. The viewing type can be the medicine dimension viewing type, the hospital dimension viewing type, the disease dimension viewing type, the doctor dimension viewing type, the medical project dimension viewing type and so on.

In particular, a correspondence relation between the viewing type sign and the dimension sign is preset, a viewing type sign is at least corresponding to two dimension signs. According to the correspondence relation, a dimension sign corresponding to the screening and viewing instruction is acquired. For example, when the viewing type sign in the screening and viewing instruction is a medicine dimension viewing type sign, then the medicine dimension viewing type sign will be corresponding to the user dimension sign and the medicine dimension sign.

Further, a node corresponding to the dimension sign and the connection relation between the acquired nodes are acquired from the treatment network diagram. A treatment network sub-diagram is generated according to the acquired nodes and the connection relations therebetween. Further, the generated treatment network sub-diagram is displayed.

Description will be made in combination with the treatment network diagram of FIG. 3. The user acquires nodes A and B corresponding to the user dimension, and nodes I, J, K and L corresponding to the medicine dimension in FIG. 3, as well as the connection relation between the user dimension corresponding nodes and the medicine dimension corresponding nodes, thereby generating the treatment network sub-diagram suitable for medicine dimension viewing type as illustrated in FIG. 5.

In the present embodiment, the screening and viewing function over the treatment network diagram is implemented, the a node corresponding to the viewing type required and the connection relations therebetween are acquired from the treatment network diagram according to the screening and viewing instruction, thereby generating the treatment network sub-diagram, i.e., screening out the partial treatment network diagram to be viewed. Which provides the viewing of the treatment network diagram flexibility and convenience, thereby identifying the suspiciously problematic treatment information more swiftly. Also, it is not necessary to display the full treatment network diagram all along, thereby saving the processing resources.

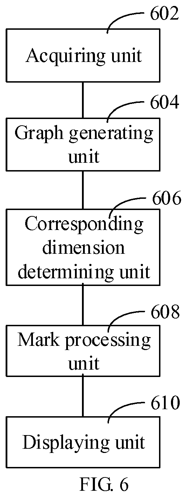

As illustrated in FIG. 6, in an embodiment, an apparatus of data displaying is provided, which includes: an acquiring unit 602, a graph generating unit 604, a corresponding dimension determining unit 606, a tag processing unit 608 and a displaying unit 610.

The acquiring unit 602 is configured to acquire an information displaying instruction which includes a displaying manner;
The acquiring unit 602 is further configured to, when the displaying manner is a first type displaying manner, acquire a first data to be processed, the first data to be processed includes treatment information in a plurality of dimensions, a dimension sign corresponding to the treatment information, and associated relations between the treatment information in different dimensions, each dimension includes at least one of treatment information.

The graph generating unit 604 is configured to generate a nodal profile according to the treatment information and the associated relations between the treatment information in different dimensions, and record the one-to-one correspondence relation between various nodes in the nodal profile and the treatment information. The connection relations between various nodes correspond to the associated relations between the treatment information in different dimensions.

The corresponding dimension determining unit 606 is configured to acquire a dimension sign corresponding to each node according to the one-to-one correspondence relation between various nodes in the nodal profile and the dimension sign corresponding to the treatment information.

The tag processing unit 608 is configured to add corresponding tag information to each node according to a preset correspondence relation between the dimension sign and the tag information, and generate a treatment network diagram.

The displaying unit 610 is configured to display the treatment network diagram.

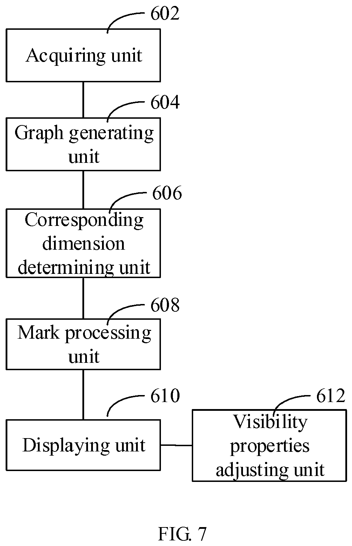

As illustrated in FIG. 7, according to an embodiment, the first data to be processed further includes the number of treatment information; In the present embodiment, the apparatus can further include:
A visibility adjustment unit 612 configured to adjust correspondingly the visibility properties of the node in the treatment network diagram and corresponding to the treatment information according to the number of treatment information, the visibility properties refer to properties that can be perceptually demonstrated.

In an embodiment, the acquiring unit 602 is further configured to, when the displaying manner is a second type displaying manner, acquire a second data to be processed, the second data to be processed includes treatment hospital information in a hospital dimension and a visit count to a treatment hospital corresponding to the treatment hospital information. The hospital dimension includes at least one of treatment hospital information.

The tag processing unit 608 is further configured to call a map application, and tag the treatment hospital corresponding to the treatment hospital information at a corresponding position on a map in the map application.

The visibility adjustment unit is further configured to adjust the visibility properties of the tag corresponding to each treatment hospital and in the map according to the visit count and generate an infographic in a geography dimension.

In an embodiment, the acquiring unit 602 is further configured to receive a screening and viewing instruction, acquire a dimension sign corresponding to the screening and viewing instruction.

The graph generating unit 604 is further configured to acquire a node corresponding to the dimension sign and the connection relation between the acquired nodes from the treatment network diagram, and generate a treatment network sub-diagram according to the acquired nodes and connection relations.

In an embodiment, the plurality of dimensions include at least two of the following: a user dimension, a medicine dimension, a medical program dimension, a hospital dimension, a disease dimension and a doctor dimension.

The various modules of the foregoing apparatus of data displaying can be implemented, in part or as a whole, by software, hardware or the combinations thereof. The foregoing modules can be embedded in or independent from the processor of a base station and in the form of hardware, or be stored in a memory of base station and in the form of software, so as to facilitate the processor to call and execute corresponding operations of the foregoing various modules. The processor can be a CPU, a microprocessor, a Single Chip Microcomputer and so on.

A person skilled in the art should understand that the processes of the methods in the above embodiments can be, in full or in part, implemented by computer programs instructing underlying hardware, the programs can be stored in a computer-readable storage medium, the program can include the processes in the embodiments of the various methods when it is being executed. The storage medium can be a disk, a CD, a Read-Only Memory (ROM) and other non-volatile storage mediums or Random Access Memory (RAM) and so on.

Although the respective embodiments have been described one by one, it shall be appreciated that the respective embodiments will not be isolated. Those skilled in the art can apparently appreciate upon reading the disclosure of the application that the respective technical features involved in the respective embodiments can be combined arbitrarily between the respective embodiments as long as they have no collision with each other.

The foregoing implementations are merely specific embodiments of the present disclosure, and are not intended to limit the protection scope of the present disclosure. It should be noted that any variation or replacement readily figured out by persons skilled in the art within the technical scope disclosed in the present disclosure shall all fall into the protection scope of the present disclosure. Therefore, the protection scope of the present disclosure shall be subject to the protection scope of the claims.

## Claims

1. A method of data displaying comprising:
acquiring an information displaying instruction, the information displaying instruction comprising a displaying manner;
acquiring a first data to be processed when the displaying manner is a first type displaying manner, the first data to be processed comprising treatment information in a plurality of dimensions, a dimension sign corresponding to the treatment information, and associated relations between the treatment information in different dimensions, wherein each dimension comprises at least one of the treatment information;
generating a nodal profile according to the treatment information and the associated relations between the treatment information in different dimensions, recording an one-to-one correspondence relation between various nodes in the nodal profile and the treatment information, wherein connection relations between various nodes correspond to the associated relations between the treatment information in different dimensions;
acquiring a dimension sign corresponding to each node according to the one-to-one correspondence relation between various nodes in the nodal profile and the dimension sign corresponding to the treatment information; and
according to a preset correspondence relation between the dimension sign and a tag information, adding corresponding tag information to each node, generating a treatment network diagram, and displaying the treatment network diagram.

2. The method of claim 1, wherein the first data to be processed further comprises the number of treatment information;
the method further comprises:
adjusting correspondingly visibility properties of the node in the treatment network diagram and corresponding to the treatment information according to the number of treatment information, wherein the visibility properties refer to properties that can be perceptually demonstrated.

3. The method of claim 1, further comprising:
acquiring a second data to be processed when the displaying manner is a second type displaying manner, wherein the second data to be processed comprises treatment hospital information in a hospital dimension and a visit count to a treatment hospital corresponding to the treatment hospital information, wherein the hospital dimension comprises at least one of treatment hospital information;
calling a map application, tagging the treatment hospital corresponding to the treatment hospital information at a corresponding position on a map in the map application; and
adjusting the visibility properties of the tag corresponding to each treatment hospital and in the map according to the visit count and generating an infographic in a geography dimension.

4. The method of claim 1, further comprising:
receiving a screening and viewing instruction, acquiring a dimension sign corresponding to the screening and viewing instruction; and
acquiring a node corresponding to the dimension sign and the connection relation between the acquired nodes from the treatment network diagram, and generating a treatment network sub-diagram according to the acquired nodes and the connection relations.

5. The method of claim 1, wherein the plurality of dimensions comprise at least two of the followings: a user dimension, a medicine dimension, a medical program dimension, a hospital dimension, a disease dimension, and a doctor dimension.

6. An apparatus of data displaying comprising:
an acquiring unit configured to acquire an information displaying instruction, the information displaying instruction comprising a displaying manner;
the acquiring unit is further configured to, acquire a first data to be processed when the displaying manner is a first type displaying manner, wherein the first data to be processed comprises treatment information in a plurality of dimensions, a dimension sign corresponding to the treatment information, and associated relations between the treatment information in different dimensions, wherein each dimension comprises at least one of the treatment information;
a graph generating unit configured to generate a nodal profile according to the treatment information and the associated relations between the treatment information in different dimensions, recording an one-to-one correspondence relation between various nodes in the nodal profile and the treatment information, wherein connection relations between various nodes correspond to the associated relations between the treatment information in different dimensions;
a corresponding dimension determining unit configured to acquire a dimension sign corresponding to each node according to the one-to-one correspondence relation between various nodes in the nodal profile and the dimension sign corresponding to the treatment information;
a tag processing unit configured to according to a preset correspondence relation between the dimension sign and the tag information, add corresponding tag information to each node, generate a treatment network diagram; and
a displaying unit configured to display the treatment network diagram.

7. The apparatus of claim 6, wherein the first data to be processed further comprises the number of treatment information;
the apparatus further comprises: a visibility adjustment unit configured to adjust correspondingly visibility properties of the node in the treatment network diagram and corresponding to the treatment information according to the number of treatment information, wherein the visibility properties refer to properties that can be perceptually demonstrated.

8. The apparatus of claim 6, wherein the acquiring unit is further configured to acquire a second data to be processed when the displaying manner is a second type displaying manner, wherein the second data to be processed comprises treatment hospital information in a hospital dimension and a visit count to a treatment hospital corresponding to the treatment hospital information, wherein the hospital dimension comprises at least one of treatment hospital information;
the tag processing unit is further configured to call a map application, tag the treatment hospital corresponding to the treatment hospital information at a corresponding position on a map in the map application; and
the visibility adjustment unit is further configured to adjust the visibility properties of the tag corresponding to each treatment hospital and in the map according to the visit count and generating an infographic in a geography dimension.

9. The apparatus of claim 6, wherein the acquiring unit is further configured to receive a screening and viewing instruction, acquire a dimension sign corresponding to the screening and viewing instruction; and
the graph generating unit is further configured to acquire a node corresponding to the dimension sign and the connection relation between the acquired nodes from the treatment network diagram, and generate a treatment network sub-diagram according to the acquired nodes and the connection relation between the acquired nodes.

10. The apparatus of claim 6, wherein the plurality of dimensions comprise at least two of the followings: a user dimension, a medicine dimension, a medical program dimension, a hospital dimension, a disease dimension and a doctor dimension.

11. A terminal comprising a processor; and a memory storing instructions, which, when executed by the processor, cause the processor to perform steps comprising:
acquiring an information displaying instruction, the information displaying instruction comprising a displaying manner;
acquiring a first data to be processed when the displaying manner is a first type displaying manner, the first data to be processed comprises treatment information in a plurality of dimensions, a dimension sign corresponding to the treatment information, and associated relations between the treatment information in different dimensions, wherein each dimension comprises at least one of treatment information;
generating a nodal profile according to the treatment information and the associated relations between the treatment information in different dimensions, recording an one-to-one correspondence relation between various nodes in the nodal profile and the treatment information, wherein connection relations between various nodes correspond to the associated relations between the treatment information in different dimensions;
acquiring a dimension sign corresponding to each node according to the one-to-one correspondence relation between various nodes in the nodal profile and the dimension sign corresponding to the treatment information; and
according to a preset correspondence relation between the dimension sign and the tag information, adding corresponding tag information to each node, generating a treatment network diagram, and displaying the treatment network diagram.

12. The terminal of claim 11, wherein the first data to be processed further comprises the number of treatment information;
the processor further executes the instructions to perform:
adjusting correspondingly visibility properties of the node in the treatment network diagram and corresponding to the treatment information according to the number of treatment information, wherein the visibility properties refer to properties that can be perceptually demonstrated.

13. The terminal of claim 11, wherein the processor further executes the instructions to provide:
acquiring a second data to be processed when the displaying manner is a second type displaying manner, the second data to be processed comprises treatment hospital information in a hospital dimension and a visit count to a treatment hospital corresponding to the treatment hospital information, wherein the hospital dimension comprises at least one of treatment hospital information;
calling a map application, tagging the treatment hospital corresponding to the treatment hospital information at a corresponding position on a map in the map application; and
adjusting the visibility properties of the tag corresponding to each treatment hospital and in the map according to the visit count and generating an infographic in a geography dimension.

14. The terminal of claim 11, wherein the processor further executes the instructions to provide:
receiving a screening and viewing instruction, acquiring a dimension sign corresponding to the screening and viewing instruction; and
acquiring a node corresponding to the dimension sign and the connection relation between the acquired nodes from the treatment network diagram, and generating a treatment network sub-diagram according to the acquired nodes and connection relations.

15. The terminal of claim 11, wherein the plurality of dimensions comprise at least two of the followings: a user dimension, a medicine dimension, a medical program dimension, a hospital dimension, a disease dimension and a doctor dimension.

16. At least one non-transitory computer-readable storage medium storing computer-readable instructions that, when executed by at least one processor, cause the at least one processor to perform steps comprising:
acquiring an information displaying instruction, the information displaying instruction comprises a displaying manner;
acquiring a first data to be processed when the displaying manner is a first type displaying manner, the first data to be processed comprises treatment information in a plurality of dimensions, a dimension sign corresponding to the treatment information, and associated relations between the treatment information in different dimensions, wherein each dimension comprises at least one of treatment information;
generating a nodal profile according to the treatment information and the associated relations between the treatment information in different dimensions, recording an one-to-one correspondence relation between various nodes in the nodal profile and the treatment information, wherein connection relations between various nodes correspond to the associated relations between the treatment information in different dimensions;
acquiring a dimension sign corresponding to each node according to the one-to-one correspondence relation between various nodes in the nodal profile and the dimension sign corresponding to the treatment information; and
adding corresponding tag information to each node according to a preset correspondence relation between the dimension sign and the tag information, generating a treatment network diagram, and displaying the treatment network diagram.

17. The non-transitory computer-readable storage medium of claim 16, wherein the first data to be processed further comprises the number of treatment information;
the processor further executes the instructions to provide:
adjusting correspondingly visibility properties of the node in the treatment network diagram and corresponding to the treatment information according to the number of treatment information, wherein the visibility properties refer to properties that can be perceptually demonstrated.

18. The non-transitory computer-readable storage medium of claim 16, wherein the processor further executes the instructions to provide:
acquiring a second data to be processed when the displaying manner is a second type displaying manner, wherein the second data to be processed comprises treatment hospital information in a hospital dimension and a visit count to a treatment hospital corresponding to the treatment hospital information, wherein the hospital dimension comprises at least one of treatment hospital information;
calling a map application, tagging the treatment hospital corresponding to the treatment hospital information at a corresponding position on a map in the map application; and
adjusting the visibility properties of the tag corresponding to each treatment hospital and in the map according to the visit count and generating an infographic in a geography dimension.

19. The non-transitory computer-readable storage medium of claim 16, wherein the processor further executes the instructions to provide:
receiving a screening and viewing instruction, acquiring a dimension sign corresponding to the screening and viewing instruction; and
acquiring a node corresponding to the dimension sign and the connection relation between the acquired nodes from the treatment network diagram, and generating a treatment network sub-diagram according to the acquired nodes and connection relations.

20. The non-transitory computer-readable storage medium of claim 16, wherein the plurality of dimensions comprise at least two of the followings: a user dimension, a medicine dimension, a medical program dimension, a hospital dimension, a disease dimension and a doctor dimension.
